Europäisches Patentamt

European Patent Office

Office européen des brevets

(11) Publication number: **0 025 873**
A1

# EUROPEAN PATENT APPLICATION

(21) Application number: 80104932.1

(22) Date of filing: 20.08.80

(51) Int. Cl.³: **A 61 K 35/78**

(30) Priority: **21.08.79 JP 105493/79**

(43) Date of publication of application:
01.04.81 Bulletin 81/13

(84) Designated Contracting States:
CH DE FR GB IT LI SE

(71) Applicant: **Aloace Co., Ltd.**
**No. 20-19 Marunouchi 2-chome Naka-ku**
**Nagoya-shi(JP)**

(72) Inventor: **Suzuki, Ikuo**
**2874-115, Minamiyama Nagasaka-cho**
**Owariasahi City Aichi-Ken(JP)**

(74) Representative: **Weitzel, Wolfgang, Dr.-Ing.**
**St. Pöltener Strasse 43**
**D-7920 Heidenheim(DE)**

(54) Anti-inflammatory agent.

(57) Aloctin A which has now been found to be useful as anti-inflammatory agent, is shown in Biochem., 85, 163-171, 1979 and Japanese Patent Application Laid Open 73109/79. However, the anti-inflammatory activity of Aloctin A has so far as is known not been heretofore described.

Croydon Printing Company Ltd.

## ABSTRACT OF DISCLOSURE

Anti-inflammatory agent containing as active component Aloctin A which is separated from *Aloe arborescens* Mill.

## BACKGROUND OF THE INVENTION

Aloctin A which has now been found to be useful as anti-inflammatory agent, is shown in Biochem., 85, 163-171, 1979 and Japanese Patent Application Laid Open 73109/79. However, the anti-inflammatory activity of Aloctin A has so far as is known not been heretofore described.

## SUMMARY OF THE INVENTION

The present invention relates to a new anti-inflammatory agent. There is a report on two lectin separated from Aloe arborescens Mill. (J. Biochem., 85, 163-171, 1979). The present inventor has found that Aloctin A (this beeing called lectin P-2 in said literature, Japanese Patent Application Laid Open No. 73109/1979) shows the excellent anti-inflammatory activity, by subsequent studies. The invention is based on this discovery.

In the present invention there can be used not only Aloctin A but also Aloctin A-containing substances, e.g., the substance which is prepared by subjecting Aloe juice to salting-out treatment, treating the resulting precipitate with alkaline aqueous solution to solubilize it, and then making the resulting solution acidic to form a precipitate (hereinafter this called Aloe acidic precipitate). This Aloe acidic precipitate shows more than 50% carragenin edema inhibition to rat at a dosage of 2-50 mg/kg by intravenous injection, and is also expected to be effective in the control of chronic inflammation. The acute toxicity of Aloctin A in mice is 400 mg/kg by subcutaneous injection, and therefore Aloctin A is a highly safe drug. The proper dose of Aloctin A for adult human varies by the cases, and is generally about 0.1-1.0 g. Administration can be done orally or subcutaneous injection, and it is also possible to apply the drug directly to skin. Suitable formulations are e.g., ointment, granule, tablet, capsule and injection. These formulations can be prepared in conventional manner using a general carrier or diluent. The invention will be illustrated in more details in the Example and Tests.

EXAMPLE: 1 kg of leaves of Aloe arborescens Mill is well crushed and the juice is filtrated with gauze. The juice is centrifuged for 30 minutes at 10,000 r.p.m. to remove contaminations and large substances. The supernatant is subjected to 0-40% ammonium sulfate fractionation, and then centrifuged for 30 minutes at 10,000 r.p.m. and obtain a precipitate. The precipitate is dissolved in 0.05 M sodium carbonate-sodium bicarbonate buffer (pH 9.5) and the resulting solution is subjected to dialysis against the same buffer to remove the salt, desalting and lyophilizing. Thus obtained precipitate is dissolved in sodium carbonate-sodium bicarbonate buffer (pH 9.5), and then a small amount of 1 M of acetic acid is added to adjest the pH to 4.4. Centrifugation is carried out for 20 minutes at 10,000 r.p.m. to obtain the precipitate. The precipitate is lyophilized to give 60 mg of Aloe acid precipitate.

TEST 1 ON THE INHIBITION OF EDEMA OF WISTAR MALE RAT

Five ml/kg of distilled water is administered orally to Wistar male rat. Each group consists of 6 male rats of 120-140 g body weight. After 30 minutes, Aloe acidic precipitate is intravenously injected at a rate of 0.5, 1, 2, 5, 10, 25, and 50 mg/kg, as a solution of isotonic sodium chloride, and then after 30 minutes 0.1 ml of 1% carragenin solution is injected to a hind paw of rat. The volume of hind paw after admistration of carragenin, is measured, and edema inhibition precentage is determined from the value before administration of carragenin. The results are shown in Table 1.

Table 1

| Dosage (mg/kg) | Edema inhibition percentage (%) | | | |
|---|---|---|---|---|
| | 1 hr. | 3 hr. | 4 hr. | 5 hr. |
| 0.5 | -3.9 | 37.4 | 36.3 | 37.1 |
| 1 | 10.0 | 33.4 | 38.2 | 25.6 |
| 2 | 24.4 | 63.9 | 62.6 | 45.5 |
| 5 | 5.0 | 53.1 | 59.5 | 51.0 |
| 10 | 6.6 | 44.8 | 51.0 | 56.2 |
| 25 | 2.3 | 83.8 | 92.0 | 96.0 |
| 50 | 1.1 | 91.6 | 92.0 | 96.0 |

Test 2 - Activity on ACII

The activity of Aloe acid precipitate on ACII (Adjuvant-Carragenin-Induced-Inflammation) is tested in accordance with the method described by Mizushima et. al,, in J. Pharm. Pharmacol., 24, 781, (1972). Each group consists of 8 female Sprague-Dawley rats of 200-240 g body weight. 0.1 ml of adjuvant and Mycobacterium butyricum (0.6 mg/0.1 ml, in liquid paraffine) are administered, and on the fifth day 10 mg/kg of Aloe acidic precipitate in physiological saline solution is administered. On the 6th day after administration of adjuvant, 0.1 ml of 1% carragenin solution is administered, and the volume of hind paw is measured at 3 and 72 hours after carragenin administration. The edema inhibiting ratio is determined from both values of hind paw of pre-and-post-administration of carragenin. Ten mg/kg of aloe acidic precipitates in physiological saline are administered at 1 hour before and at 24 hours after administration of carragenin. Therefore, administration of the Aloe acid precipitate is carried out three times. Similar test is performed using cyclophosphamide as control. The results are shown in Table 2.

Table 2

| Drugs | Dossage (mg/kg) | Edema inhibition percentage (%± S.E.) | |
| --- | --- | --- | --- |
| | | Acute Phase | Delayed Phase |
| Aloe acid precipitate | 10 | 62.8± 7.3 [*3] | 39.7± 5.9 [*1] |
| Cyclophosphami-de (control) | 10 | 13.6± 3.0 [*1] | 50.0± 2.5 [*2] |

[*1] P < 0.05  [*2] P < 0.01  [*3] P < 0.001

CLAIMS

1. Anti-inflammatory agent containing as active component Aloctin A.

2. A anti-inflammatory composition containing an essential active ingredient Aloctin A and a carrier therefor.

**0025873**

European Patent Office

**EUROPEAN SEARCH REPORT**

Application number

EP 80 10 4932

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | EP - A - 0 002 452 (ALOACE Co. Ltd.)<br>* Page 14, line 1 to page 22, line 13 * | 1,2 | A 61 K 35/78 |
| D | & JP - A - 73109/79 | | |
| | -- | | |
| XD | CHEMICAL ABSTRACTS, vol.90, nr.15, April 9, 1979, page 473, abstract 119532y Columbus, Ohio. USA SUZUKI,I. et al. "Purification and characterization of two lectins from Aloe arborescens Mill".<br>& J. Biochem. (Tokyo), 1979, 85(1), 163-71.<br>* Abstract * | 1,2 | |
| | -- | | TECHNICAL FIELDS SEARCHED (Int. Cl.³)<br><br>A 61 K 35/78 |
| A | FR - A - 2 044 371 (MARSH JOSEPH ROGERS Jr)<br>* Page 2, lines 16-35 * | 1,2 | |
| | -- | | |
| A | US - A - 3 892 853 (HENRY H. COBBLE)<br>* Column 6, lines 21-63 * | 1,2 | |
| | -- | | |
| A | US - A - 3 920 816 (MANFRED I.L. SEEGALL et al. )<br>* Column 3, line 26 to column 8, line 14 * | 1,2 | CATEGORY OF CITED DOCUMENTS |
| | ---- | | X: particularly relevant<br>A: technological background<br>O: non-written disclosure<br>P: intermediate document<br>T: theory or principle underlying the invention<br>E: conflicting application<br>D: document cited in the application<br>L: citation for other reasons |

&: member of the same patent family, corresponding document

The present search report has been drawn up for all claims

| Place of search The Hague | Date of completion of the search 13-01-1980 | Examiner REMPP |
|---|---|---|

EPO Form 1503.1   06.78